# FASCICULE DE BREVET EUROPEEN

(11) **EP 2 579 781 B1**
(45) Date de publication et mention de la délivrance du brevet: **27.02.2019**
(21) Numéro de dépôt: 11720484.2
(22) Date de dépôt: 19.05.2011
(51) Int. Cl.: A61B 10/02, A01K 11/00

(54) **UNITÉ DE PRÉLÈVEMENT DE MATÉRIEL ANIMAL**
EINHEIT ZUR PROBENAHME VON TIERMATERIAL
UNIT FOR SAMPLING ANIMAL MATERIAL

(30) Priorité: 09.06.2010 FR 1054564
(43) Date de publication de la demande: 17.04.2013
(73) Titulaire: Allflex Europe, 35500 Vitré (FR)
(72) Inventeur: DECALUWE, Johan, F-53000 Laval (FR); DE MEULEMEESTER, Johan, B-9840 De Pinte (BE)
(74) Mandataire: Vidon Brevets & Stratégie
(86) Numéro de dépôt international: PCT/EP2011/058106
(87) Numéro de publication internationale: WO 2011/154233

(56) Documents cités:
- EP-A2- 1 504 722
- WO-A2-02/078431
- US-A- 5 902 280
- US-A1- 2007 239 067
- US-A1- 2008 227 662
- US-B1- 6 659 338

## Description

### 1. Domaine de l'invention

Le domaine de l'invention est celui du contrôle d'animaux, ou de produits issus d'animaux, par le biais d'un prélèvement d'un matériel animal.

Par matériel animal, on entend ici tout matériel pouvant être prélevé sur un animal vivant ou mort, éventuellement après transformation, comme un échantillon de tissu, un échantillon sanguin, un échantillon de moelle osseuse, un échantillon de viscère, une portion de l'animal destiné à la consommation, un morceau de cuir, etc.

Plus précisément, l'invention concerne les moyens de réalisation d'un tel prélèvement et leur conditionnement.

### 2. Art antérieur

Il existe de nombreuses occasions où il est nécessaire d'effectuer un prélèvement sur un matériel animal.

Il peut s'agir de prélèvements exigés par des évènements particuliers de la vie d'un animal tels que sa naissance, la menace d'une maladie, son transport ou son abattage, ou encore de prélèvements périodiques, ou bien encore de prélèvements effectués après la mort de l'animal, par exemple sur une portion d'un animal destinée à la consommation ou sur du cuir.

Ces prélèvements peuvent être réalisés par les éleveurs eux-mêmes, ou par des intervenants ponctuels, comme des services vétérinaires, le personnel d'un abattoir ou des personnes effectuant un contrôle qualité pour une entreprise de distribution agro-alimentaire.

Pour éviter des risques d'infection de l'animal prélevé ou d'altération du matériel prélevé, il est nécessaire de disposer d'un matériel aseptisé lors de ces prélèvements. Ceci inclut non seulement les moyens de perforation du matériel animal, en contact direct avec l'animal et/ou le matériel animal, mais également l'ensemble des outils ou moyens utilisés lors du prélèvement, par exemple les pinces ou seringues, qui doivent être désinfectés régulièrement.

Cette contrainte est d'autant plus importante pour les personnes intervenant ponctuellement sur plusieurs cheptels. Il est en effet primordial qu'elles n'introduisent pas dans un cheptel une maladie en provenance d'un autre cheptel précédemment visité du fait d'une mauvaise désinfection d'un outil.

Il faut également faire attention à ne pas contaminer les outils de prélèvement, et en
particulier les éléments permettant de perforer le matériel animal, lors de leur manipulation.

Ceci est d'autant plus vrai lorsque la personne qui effectue le prélèvement n'a pas une longue habitude des prélèvements et/ou lorsque le prélèvement est réalisé sur un animal vivant, et donc susceptible de bouger lors du prélèvement.

Il est également important de limiter les risques de blessure de l'intervenant lors du prélèvement.

Pour tenter de résoudre tout ou partie des problèmes cités, il est connu du document US6659338, qui représente l'état de la technique le plus proche, un dispositif de prélèvement selon lequel les échantillons biologiques prélevés sont placés dans des capsules fermées.

Par ailleurs, il est connu du document US2008227662 un système de réception d'échantillons de tissus et d'identification des sources des échantillons.

En outre, il est connu du document US2007239067 un dispositif de biopsie selon lequel l'échantillon de tissu coupé est sélectivement disposé dans un conteneur préalablement enregistré par le dispositif.

Il est également connu du document WO02078431 une paire de pinces pour étiquettes d'oreille pour animaux permettant, en outre, de prélever une partie de tissu de l'oreille.

Il est connu du document EP1504722 un outil de prélèvement d'échantillons, visant à éviter les risques de contamination de l'échantillon prélevé.

Il est connu du document US5902280 un outil de biopsie comprenant une aiguille apte à collecter des cellules par aspiration lorsque l'aiguille coopère avec un conteneur sous vide.

Néanmoins, quelque soit la solution retenue, celle-ci est relativement complexe à utiliser et n'offre pas de garantie en termes de sécurité sanitaire et également en terme de sécurité des intervenants.

Il existe donc un besoin pour un matériel de prélèvement simple à utiliser, offrant des garanties en termes de sécurité sanitaire et également en terme de sécurité des intervenants.

### 3. Exposé de l'invention

L'invention propose une solution nouvelle qui ne présente pas l'ensemble de ces inconvénients de l'art antérieur, sous la forme d'une unité de prélèvement d'au moins un échantillon de matière animale, formée d'un emballage comprenant au moins un ensemble de prélèvement, telle que définie dans la revendication indépendante 1. Plusieurs caractéristiques particulières de l'invention sont définies dans les revendications dépendantes.

Selon l'invention, un tel ensemble de prélèvement comprend notamment :
- un outil de prélèvement ;
- un élément de prélèvement dudit échantillon ;
- un élément de stockage dudit échantillon ;
au moins un desdits éléments de prélèvement ou de stockage étant pré-monté sur au moins un support prévu à cet effet sur ledit outil de prélèvement.

La solution de l'invention offre ainsi, à un éleveur ou à tout autre utilisateur, l'avantage d'un dispositif « clé en main », simple d'utilisation.

De plus, la solution permet un usage unique de l'outil de prélèvement, ce qui garantit une bonne hygiène de l'outil de prélèvement.

Ledit ensemble de prélèvement est destiné au prélèvement d'un matériel animal quelconque, vivant ou inerte, par exemple un tissu animal comme un morceau d'oreille, de peau, de viscère, ou un fluide animal comme de la moelle osseuse ou du sang.

L'ensemble de prélèvement de l'invention peut notamment offrir la possibilité disposer de plusieurs supports de positionnement.

Selon l'invention, au moins un support définit une position de prélèvement pour ledit élément de prélèvement.

La solution de l'invention offre ainsi l'avantage, grâce à une position pré-montée dudit élément de prélèvement dans sa position de prélèvement, de limiter les erreurs de manipulation lors de la préparation du prélèvement, qui pourraient occasionner une blessure de l'utilisateur ou nuire à la qualité du prélèvement, du fait d'une mauvaise installation d'un élément.

Enfin la présence d'un support de transport après réalisation du prélèvement, permet à la personne en charge du prélèvement d'avoir à sa disposition un moyen de rangement pratique de l'échantillon prélevé et de limiter ainsi les risques de perte ou d'altération de l'échantillon.

Selon une caractéristique particulière de l'invention, au moins un desdits supports comprend une saillie permettant de pousser ledit matériel animal dans ledit élément de stockage et/ou de prélèvement après prélèvement dudit matériel animal.

Selon une caractéristique particulière de l'invention, ledit outil de prélèvement est formé d'un seul tenant.

La solution de l'invention permet ainsi de proposer un outil simple à réaliser et de faible coût de fabrication.

L'outil de prélèvement peut notamment être réalisé en matière plastique, en moulage par injection par exemple.

Selon une caractéristique particulière de l'invention, ledit élément de prélèvement est pré-monté sur ledit support dans une position de prélèvement, de façon irréversible.

Ainsi, la solution offre l'avantage de n'autoriser qu'un usage unique de l'outil de prélèvement. Lorsque le prélèvement est réalisé par un tiers, par exemple un vétérinaire, elle permet donc de garantir à l'éleveur une bonne hygiène de l'outil de prélèvement.

De plus, la solution de l'invention offre aussi l'avantage de diminuer le nombre d'opérations nécessaires à la réalisation d'un prélèvement dans certains modes de réalisation. Elle permet ainsi de proposer à un utilisateur un fonctionnement plus simple dudit outil. Elle permet également de diminuer la complexité de l'outil de prélèvement.

Selon une caractéristique particulière de l'invention, ledit élément de prélèvement est logé de façon amovible dans ledit élément de stockage, de façon à protéger ledit élément de prélèvement.

Ainsi, la solution de l'invention permet d'éviter que l'élément de prélèvement puisse être altéré, par exemple tordu, lors du transport de l'unité de prélèvement ou puisse blesser la personne qui le transporte ou l'installe, notamment un éleveur qui transporterait une telle unité de prélèvemenent dans une poche de vêtement.

Elle permet également de conserver l'élément de prélèvement dans un environnement propre.

Selon l'invention, ledit élément de prélèvement comprend un élément de coupe, destiné à découper ledit matériel animal, et un élément poussoir, permettant de pousser ledit matériel animal dans ledit élément de stockage après découpe dudit matériel animal par ledit élément de coupe.

Ainsi, il est possible de ne conserver que l'élément de stockage, une fois le prélèvement réalisé.

Ce mode de réalisation offre de plus l'avantage de réaliser une obturation de l'élément de stockage sans nécessité une intervention de l'utilisateur.

Selon une caractéristique particulière de l'invention, au moins un desdits éléments de prélèvement et/ou de stockage contient au moins un agent spécifique appartenant au groupe comprenant :
- un agent désinfectant ;
- un agent cicatrisant ;
- un agent conservateur ;
- un agent dessiccant ;
- un médicament ;
- un vaccin ;
- une combinaison d'au moins deux des agents précédents.

Ainsi, l'élément de prélèvement ou de stockage peut éventuellement comprendre des moyens de traitement de l'échantillon permettant sa préparation, sa conservation, ou encore son analyse.

Si l'agent est un agent désinfectant ou cicatrisant, il permet de détruire au moins en partie les micro-organismes (bactéries, virus, etc) pouvant contaminer l'échantillon prélevé.

Si l'agent est un agent conservateur ou dessicant, il permet d'améliorer la conservation d'un échantillon de tissu à prélever, et éventuellement de le préparer en vue d'un traitement ultérieur comme une analyse ADN.

Si l'agent est un médicament ou un vaccin au contact de l'élément de prélèvement, il permet de soigner ou traiter l'animal en cas de besoin, cet agent pouvant passer dans le sang au niveau des tissus perforés ou être absorbé par la peau de l'animal.

Selon une caractéristique particulière de l'invention, ledit agent spécifique se présente sous l'une des formes appartenant au groupe comprenant :
- un gel ;
- une crème ;
- une graisse,
- un liquide ;
- une poudre ;
- un gaz ;
- une mousse imprégnée.

Selon une caractéristique particulière de l'invention, au moins un élément de prélèvement consiste en une aiguille de biopsie , un emporte-pièce, ou un poinçon.

Selon une caractéristique particulière de l'invention, ledit outil de prélèvement comprend des moyens de fermeture dudit élément de stockage.

La solution de l'invention propose ainsi une meilleure fermeture des moyens de stockage : en effet, comme la fermeture est réalisée automatiquement, il ne peut y avoir de risque d'oubli, de plus, les moyens de fermeture étant adaptés aux moyens de stockage, le résultat est en général plus satisfaisant que lors d'une opération manuelle, où l'opérateur n'osera pas par exemple appuyer trop fortement sur un bouchon, de risque de faire éclater un tube en verre.

Dans certains modes de réalisation où les moyens de fermeture permettent d'empêcher un accès audit élément de stockage et/ou audit matériel animal prélevé, sans détérioration visible dudit outil, la solution permet de garantir que le matériel animal prélevé par l'outil sera bien le matériel animal qui parviendra au laboratoire, en cas de manipulation par des tiers.

Elle permet également d'apporter une protection supplémentaire à l'échantillon lors du transport, notamment en cas de suspicion de risques épidémiologiques.

Selon l'invention, ledit outil de prélèvement est une pince. En outre, ledit outil de prélèvement comprend un moyen de retour, permettant de ramener l'outil de prélèvement dans une position initiale une fois le prélèvement réalisé.

On entend par position initiale la position dudit outil antérieurement au prélèvement.

Ainsi, la solution de l'invention offre l'avantage de libérer plus rapidement le matériel animal sur lequel réaliser le prélèvement, ce qui est particulièrement utile lorsqu'il s'agit d'un animal vivant, pour qui le prélèvement constitue une agression.

Le moyen de retour peut notamment être constitué d'au moins une lame ressort.

Selon un mode de réalisation particulier de l'invention, ledit outil de prélèvement comprend au moins deux supports, et au moins un desdits supports est destiné à porter au moins un élément de prélèvement et/ou de stockage supplémentaire.

Ainsi la solution offre par exemple l'avantage à un intervenant de pouvoir utiliser le second élément de prélèvement et/ou de stockage si le premier élément de prélèvement et/ou de stockage est souillé ou altéré avant la réalisation du prélèvement, par exemple par une chute.

Elle permet également de réaliser plusieurs prélèvements distincts, dans le but de faire des analyses distinctes ou parce qu'une analyse nécessite plusieurs prélèvements, notamment du fait qu'elle met en oeuvre des agents spécifiques différents.

Par exemple, la solution permet un double prélèvement comportant un premier prélèvement utilisant un agent cicatrisant puis un second prélèvement utilisant un autre agent.

Selon une caractéristique particulière de l'invention, ledit outil de prélèvement est, au moins partiellement, recyclable.

Ainsi, la solution de l'invention offre des avantages en terme de développement durable.

Selon une caractéristique particulière de l'invention, ledit outil de prélèvement est, au moins partiellement, conçu dans un matériau permettant un usage unique dudit outil.

Par usage unique, on entend que le mode de fabrication et/ou de délivrance de l'outil de prélèvement est adapté pour que l'outil de prélèvement puisse être utilisé une seule fois. Cependant, certains modes de réalisation de la pince rendent possible que l'outil soit utilisé un nombre limité de fois (par exemple de l'ordre de vingt-cinq utilisations).

Selon une caractéristique particulière de l'invention, ledit élément de stockage contient des moyens anti-retour, permettant de retenir ledit matériel animal dans ledit élément de stockage lors de son ouverture.

Selon un mode de réalisation particulier de l'invention, ledit outil de prélèvement, ledit élément de stockage, ledit élément de prélèvement et/ou ledit emballage comprennent des moyens d'identification permettant d'identifier ledit ensemble de prélèvement et/ou ladite unité de prélèvement.

Ladite unité de prélèvement peut, dans certains modes de réalisation où elle ne contient qu'un seul ensemble de prélèvement, posséder la même identification que l'ensemble de prélèvement lui-même. Dans d'autres modes de réalisation, par exemple des modes de réalisation dans lesquels l'unité de prélèvement comporte plusieurs ensembles de prélèvement, l'unité de prélèvement et au moins un ensemble de prélèvement qu'elle contient peuvent éventuellement avoir des identifications distinctes. Dans ce cas, l'emballage de l'unité de prélèvement peut par exemple comporter l'identification de l'unité de prélèvement ainsi que celles des ensembles de prélèvement qu'il contient.

Il peut s'agir de moyens d'identification visuels, sous forme d'une séquence de chiffre ou d'un code barre, éventuellement 2D par exemple, ou électronique, sous forme d'une puce RFID notamment.

Ainsi, la solution permet d'identifier, pour au moins une composante d'une unité de prélèvement, l'unité de prélèvement et/ou l'ensemble de prélèvement dont elle fait partie et donc de garder une traçabilité entre certaines composantes,voire toutes, d'une même unité de prélèvement.

Dans certains modes de réalisation, la solution de l'invention permet à un utilisateur d'associer, au moment du prélèvement, l'identification de l'unité de prélèvement et/ou de l'ensemble de prélèvement à l'identification de la matière animale prélevée, par exemple par le biais d'un enregistrement dans une base de données centralisée.

Selon une caractéristique particulière de l'invention, lesdits moyens d'identification peuvent contenir des moyens d'identification de ladite matière animale prélevée.

Ainsi la solution permet notamment d'associer dès sa fabrication une unité de prélèvement à au moins un animal.

Selon une caractéristique particulière de l'invention, ledit emballage est stérile.

Un tel emballage permet ainsi la protection d'un ensemble de prélèvement selon l'invention. Notamment, il permet sa conservation dans un environnement stérile. De ce fait, il permet de garder stérile un ensemble de prélèvement depuis sa fabrication jusqu'à son utilisation, même s'il est manipulé sans précaution. Il peut également lui apporter une isolation thermique, tout spécialement si l'ensemble de prélèvement comprend un élément de prélèvement ou de stockage qui a été pré-rempli avec un agent spécifique. Enfin, un tel emballage permet de préserver l'ensemble de prélèvement des chocs ou des contacts avec d'autres matériels.

Selon une caractéristique particulière de l'invention, ledit emballage comprend en outre au moins un élément de prélèvement ou de stockage supplémentaire.

### 4. Liste des figures

D'autres caractéristiques et avantages de l'invention apparaîtront plus clairement à la lecture de la description suivante de deux modes de réalisation particuliers, donnés à titre de simples exemples illustratifs et non limitatifs, et des dessins annexés, parmi lesquels :

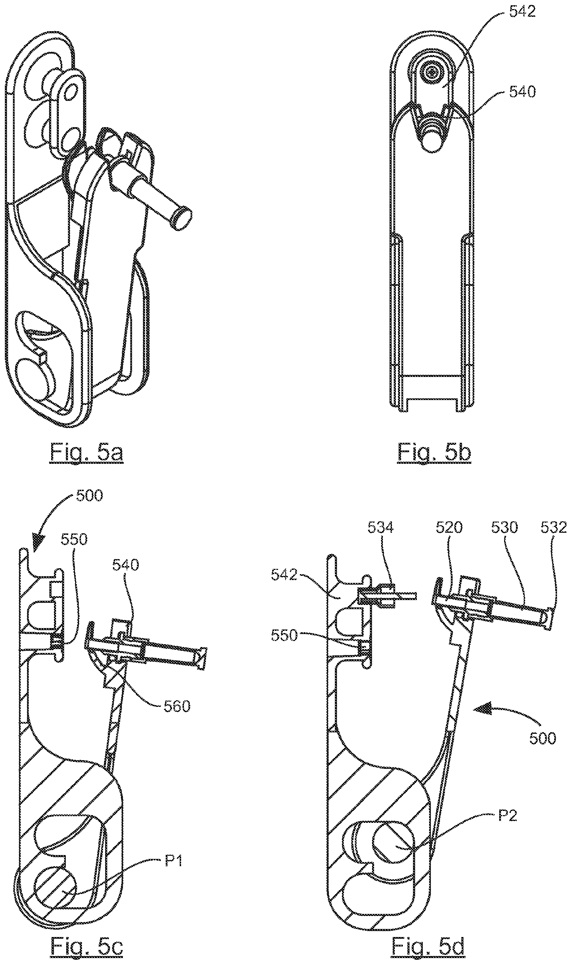

- les figures 5a à 5d, d'une part, et 6a et 6b, d'autre part, illustrent respectivement un ensemble de prélèvement selon deux modes de réalisation de l'invention ;
- les autres figures illustrent d'autres exemples d' ensembles de prélèvement qui ne font toutefois pas partie de la présente invention telle que définie par les revendications, mais qui sont utiles pour la compréhension de la présente invention.

### 5. Description d'un mode de réalisation de l'invention

### 5.1 Principe général

Le principe général de l'invention consiste à proposer à une personne en charge de la réalisation de prélèvements, par exemple un éleveur, un vétérinaire, un employé d'un abattoir ou d'une usine de transformation agro-alimentaire, etc, une unité de prélèvement comportant, dans un emballage, au moins un ensemble de prélèvement stérile, « tout-en-un », lui permettant de disposer de l'ensemble des moyens nécessaires à la réalisation d'au moins un prélèvement sur une matière animale et de réaliser très simplement ce prélèvement. Un tel ensemble de prélèvement comprend notamment un outil de prélèvement (tel qu'une pince), un élément de prélèvement (tel qu' un emporte-pièce), un élément de stockage (tel qu'un tube d'échantillonnage) du matériel prélevé. Selon l'invention, l'élément de prélèvement est pré-monté sur l'outil de prélèvement, lors de la fabrication dudit ensemble.

Selon un mode particulier de réalisation, l'ensemble de prélèvement est prévu pour la réalisation d'un unique prélèvement. Ce mode de réalisation permet de prévenir la contamination de l'outil de prélèvement lors d'un prélèvement précédent. Une fois utilisé, l'outil de prélèvement peut être aussitôt jeté par l'utilisateur ou conservé comme moyen de transport de l'échantillon puis jeté par un tiers. L'outil de prélèvement peut notamment être conçu de façon à être recyclable.

Certains modes de réalisation de l'ensemble de prélèvement rendent possible une réutilisation de l'outil. Cependant, l'outil n'étant pas conçu pour résister à l'usure, cette réutilisation ne sera possible que de façon limitée, par exemple de l'ordre de vingt-cinq utilisations.

Du fait qu'un tel ensemble est destiné à un nombre limité de prélèvements, il offre l'avantage de ne pas nécessiter d'entretien.

Ceci induit une simplification du point de vue de l'utilisateur, mais également une simplification du mode de fabrication, les pièces n'ayant plus besoin d'être démontables par exemple.

Encore un avantage de l'invention, du fait de l'absence de contrainte de résistance à l'usure, est l'utilisation d'un matériau plus léger, et/ou de dimension plus faible, que les solutions de l'art antérieur, ce qui permet de proposer un outil plus léger, donc plus facile à utiliser. Par exemple, l'outil de prélèvement peut être réalisé en plastique.

Encore un avantage de l'invention, du fait du matériel utilisé et de la simplicité induite dans la réalisation de l'outil de prélèvement, est le faible coût de fabrication de l'unité de prélèvement. Ce faible coût rend possible à un utilisateur de changer fréquemment d'outil de prélèvement, parce que le précédent n'est plus utilisable ou parce qu'il veut disposer d'un modèle plus récent.

Au contraire, les outils de prélèvement de l'art antérieur sont souvent onéreux, du fait de leur complexité et des matériaux mis en oeuvre, ce qui pousse un éleveur à posséder une unique pince, qui peut être partagée par plusieurs utilisateurs, augmentant ainsi les problèmes d'hygiène.

L'invention, par contre, donne à un éleveur la possibilité d'acquérir plusieurs pinces, chaque pince étant attribuée par exemple à un utilisateur particulier.

L'invention permet également de ce fait un gain de temps important, notamment lorsqu'il est nécessaire de réaliser un prélèvement sur chaque tête d'un cheptel de taille importante, puisque plusieurs prélèvements peuvent être réalisés en même temps. Cette rapidité de prélèvement peut de plus induire une rapidité de diagnostic en cas d'épisode épidémiologique.

L'un des avantages de la solution de l'invention découle également, dans certains modes de réalisation, de l'absence de moyens de marquage (permettant la pose d'une marque d'identification) simultané.

En effet, l'ensemble de prélèvement selon au moins un mode de réalisation de l'invention offre l'avantage de pouvoir être utilisé sur un animal possédant déjà une boucle d'identification. En effet, si l'animal est déjà marqué, le prélèvement d'un échantillon de matière animale en utilisant un dispositif effectuant simultanément l'identification et le prélèvement résulterait en l'attribution d'un nouvel identifiant à un même animal. La solution de l'invention offre au contraire l'avantage à un éleveur de disposer d'une solution permettant des analyses répétées sur un même animal.

Un autre avantage du dispositif de l'invention est son adaptation à un échantillonnage ADN. En effet, les analyses ADN sont coûteuses. Un éleveur n'a donc pas intérêt à faire cette analyse à la naissance de l'animal (où il doit être identifié) mais lorsque l'animal se révèle viable et lorsque l'éleveur sait à quel usage il va l'affecter.

### 5.2 Exemples d'unités de prélèvement

Une unité de prélèvement selon l'invention est formée d'un emballage, comprenant au moins un ensemble de prélèvement.

On décrit ci-après, en relation avec les figures la à 1d, un premier ensemble de prélèvement utilisé par exemple pour le prélèvement d'un échantillon d'oreille d'un animal.

Ainsi, comme illustré par la figure la, un tel ensemble de prélèvement 100 comprend un outil de prélèvement, qui peut notamment prendre la forme d'une pince 11.

Il comprend également un élément de prélèvement 120 et un élément de stockage 130, ainsi que des supports 140, 142 et 144 situés sur l'outil de prélèvement, destinés à porter l'élément de prélèvement 120 et/ou l'élément de stockage 130.

### 5.2.1 Elément de prélèvement

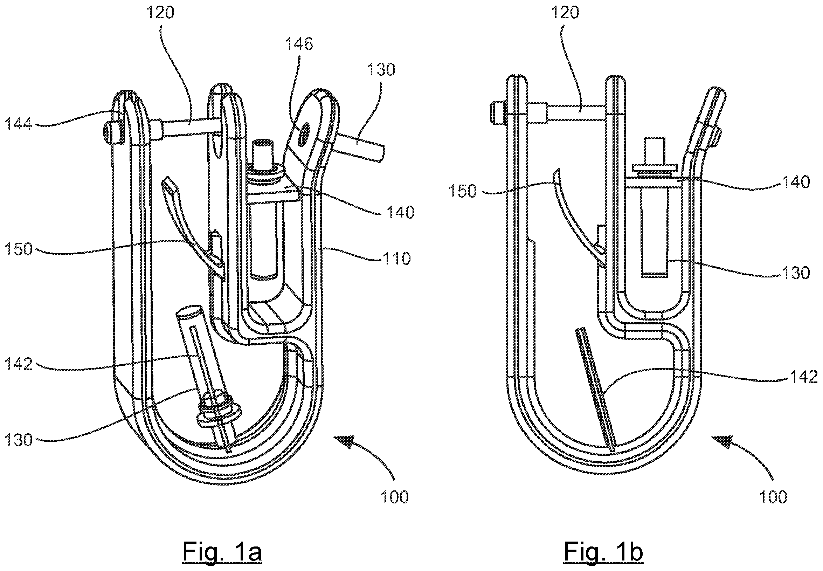

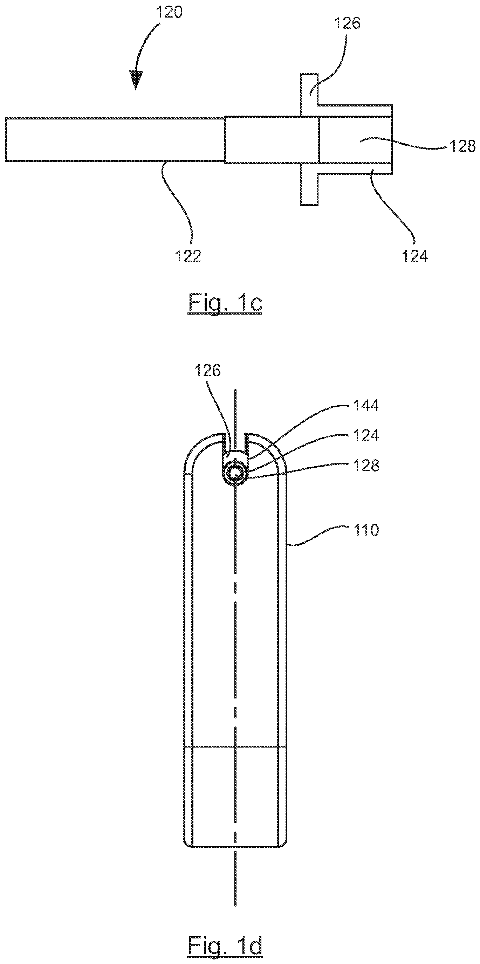

On présente en relation avec la figure 1c, une vue de coupe de l'élément de prélèvement 120.

Celui-ci comprend un élément de coupe présentant une arête coupante.

Dans le mode de réalisation présenté, l'élément de coupe consiste en un emporte-pièce 122, monté sur un support rigide et creux 124, qui peut être réalisé en matière plastique, comportant une collerette périphérique 126.

Le support 124 comprend une cavité 128, destinée à coopérer avec un pointeau de l'outil de prélèvement 120.

Si l'élément de coupe est inséré dans l'élément de stockage 130 (antérieurement ou postérieurement au prélèvement), la collerette 126 de l'élément de coupe 122 peut servir de butée pour limiter la couverture de l'élément de coupe 122 par l'élément de stockage 130, et ainsi aménager un espace entre l'extrémité de l'élément de stockage 130 et l'élément de coupe 122.

Cet espace peut notamment permettre de loger l'échantillon prélevé dans l'élément de stockage 130.

Selon une variante, l'élément de coupe 122 et le support 124 sont formés d'une seule pièce, par exemple en plastique ou en métal.

Selon une autre variante, un tel élément de prélèvement peut également comprendre un élément poussoir mobile par rapport à l'élément de coupe, tel que décrit dans la demande de brevet français déposée sous le numéro FR-08 58453, non publiée, permettant de pousser le matériel animal dans l'élément de stockage après découpe.

### 5.2.2 Elément de stockage

Comme illustré en figure la, l'élément de stockage 130 consiste par exemple en un tube apte à contenir au moins partiellement l'élément de prélèvement, de façon à recouvrir l'extrémité coupante de l'emporte-pièce et à isoler le matériel animal prélevé de l'environnement extérieur.

L'élément de prélèvement 120 ferme alors de façon étanche ou quasi-étanche le tube de stockage 130.

Selon une variante, le matériel animal est poussé dans l'élément de stockage après prélèvement. L'élément de stockage peut alors être fermé par un bouchon, ou par un élément de l'outil de prélèvement, ou par l'élément poussoir décrit ci-dessus, etc.

En particulier, l'élément de stockage peut comprendre une tête de tube, comprenant une collerette, prenant appui sur le rebord du tube de stockage. Il peut également contenir des moyens de rétention permettant de conserver le matériel animal prélevé dans l'élément de stockage lors de son ouverture.

### 5.2.3 Outil de prélèvement

Dans certains modes de réalisation, l'outil de prélèvement 110 est, au moins partiellement, conçu dans un matériau permettant un usage unique de l'outil 110. En particulier, le mode de réalisation illustré en figure la prévoit que l'outil de prélèvement 110 est constitué d'une pièce unique, obtenue par exemple en moulage par injection d'un matériau plastique. L'outil de prélèvement 110 peut être, au moins partiellement, recyclable.

Dans le mode de réalisation décrit en figure la, l'outil de prélèvement 110 est formé d'un seul tenant et se présente sous la forme d'une pince comprenant deux branches ou poignées quelconques.

Une pression, manuelle par exemple, sur les branches, permet de guider l'élément de prélèvement pour qu'il pénètre la chair de l'animal et force l'entrée de la matière animale prélevée dans l'élément de stockage.

La pince 110 peut également être actionnée au moyen d'une énergie électrique, pneumatique, ou autre. L'outil de prélèvement 110 comprend au moins un support destiné au positionnement de l'élément de prélèvement 120 et/ou de l'élément de stockage 130. Ces supports peuvent notamment permettre un positionnement de l'élément de prélèvement 100 ou de l'élément de stockage 130 dans diverses positions :
- une position initiale de transport, du moment de la fabrication de l'ensemble de prélèvement jusqu'à la réalisation du prélèvement,
- une positionnement de prélèvement, ou encore
- une position finale de transport, après la réalisation du prélèvement jusqu'à son acheminement dans un laboratoire par exemple.

Par exemple, la figure la illustre les différentes positions des éléments de prélèvement ou de stockage mises en oeuvre successivement :
- avant que soit emballé l'ensemble de prélèvement pour former l'unité de prélèvement, l'élément de prélèvement 120 peut être inséré dans l'élément de stockage 130, maintenu dans un premier support 140 de l'outil de prélèvement 110, prenant par exemple la forme d'une lumière dans laquelle vient s'insérer l'élément de stockage 130. Ce premier support 140 définit une position initiale de transport ;
- lors du prélèvement, l'élément de prélèvement 120 est positionné sur une première mâchoire de l'outil, dans un deuxième support 144, et l'élément de stockage 130 est positionné sur une deuxième mâchoire de l'outil, dans un troisième support 146. Ces deux supports 144 et 146 définissent une position de prélèvement ;
- après prélèvement, l'élément de prélèvement 120 peut être inséré dans l'élément de stockage 140, et maintenu sur un quatrième support 142, prenant par exemple la forme d'une tige. Une telle tige peut notamment venir s'insérer dans l'élément de prélèvement par son extrémité opposée à l'arête coupante, au niveau de la cavité 128, et venir pousser le matériel prélevé dans l'élément de stockage. La cavité 128 peut par exemple être obturée grâce à un matériau perméable de type mousse ou encore percée d'une ouverture étroite. Ce quatrième support 142 définit une position finale de transport.

Ces positions peuvent être distinctes, comme illustré par les supports 140, 142, 144 et 146 de la figure la, ou confondues.

En particulier, les positions initiale et finale de transport peuvent être confondues.

Selon une variante, les éléments de prélèvement et de stockage sont directement pré-montés dans la position de prélèvement avant que soit emballél'ensemble de prélèvement pour former l'unité de prélèvement.

Différents modes de solidarisation des éléments de prélèvement ou de stockage avec le support peuvent être envisagés.

Par exemple, dans le mode de réalisation illustré en figure 1d, l'élément de prélèvement 120 est solidarisé au deuxième support 144 dans la position de prélèvement grâce à sa collerette 126.

Il est également prévu qu'au moins un des supports comprend une saillie permettant de pousser le matériel animal dans l'élément de stockage et/ou dans l'élément de prélèvement après prélèvement du matériel animal lorsque l'élément de stockage et/ou dans l'élément de prélèvement est positionné sur le support. Il s'agit par exemple de la tige du quatrième support 142 illustrée en figures la et 1b, qui permet notamment de pousser l'échantillon prélevé au fond du tube de stockage.

La présence d'une telle saillie sur un support peut également permettre de fermer un élément de stockage positionné sur ce support.

Dans une variante du mode de réalisation présenté par les figures la à 1d, l'outil de prélèvement comprend plusieurs supports destinés à porter au moins un élément de prélèvement ou de stockage supplémentaire, de façon par exemple à permettre un remplacement de l'élément de prélèvement ou de stockage en cas de défaut ou de souillure. Ainsi, plusieurs éléments de prélèvement et/ou de stockage peuvent être présents simultanément sur l'outil de prélèvement.

Ces éléments de prélèvement et/ou de stockage multiples peuvent également permettre de réaliser plusieurs prélèvements avec un même outil de prélèvement, par exemple pour une analyse nécessitant plusieurs prélèvements ou pour des analyses distinctes.

L'outil de prélèvement 110 comprend en outre un moyen de retour, permettant de ramener l'outil de prélèvement dans sa position initiale une fois le prélèvement réalisé. Ce moyen de retour peut par exemple consister en un ressort ou, comme présenté en figure la, en une patte flexible 150.

Dans un mode de réalisation particulier, compatible avec le mode de réalisation présenté en figure la, l'élément de prélèvement et/ou l'élément de stockage peut contenir au moins un agent spécifique visant à la préparation, la conservation, ou encore le traitement de l'échantillon.

Il peut par exemple s'agir d'un agent désinfectant, cicatrisant ou séchant, comme un silicagel (marque déposée) ou plus généralement un tamis moléculaire à base de silice, d'argile, ou autre, d'un agent conservateur, d'un médicament ou d'un vaccin ou encore d'une combinaison d'au moins deux des agents précédents.

Différents types de produit se présentant sous la forme de billes, de poudre, de gel, de crème, de graisse, d'un liquide, d'une crème , d'un gaz etc, éventuellement imprégnés dans une mousse, peuvent être utilisés.

En d'autres termes, l'agent spécifique selon ce mode de réalisation peut prendre la forme de tout produit pouvant agir sur le matériel animal sur lequel est effectué le prélèvement, ou sur l'échantillon prélevé.

### 5.2.4 Unité de prélèvement

L'ensemble de prélèvement 100 est conditionné dans un emballage, formant ainsi une unité de prélèvement.

L'emballage de l'unité de prélèvement et/ou l'ensemble de prélèvement 100 lui même peuvent comprendre des moyens d'identification. Il peut s'agir de moyens d'identification visuels, sous forme d'une séquence de chiffre ou d'un code barre, éventuellement 2D par exemple, ou électronique, sous forme d'une puce RFID notamment.

Par exemple, on affecte un même numéro d'identification à l'outil de prélèvement, à l'élément de prélèvement, à l'élément de stockage, et/ou à l'emballage. Ces moyens d'identification permettent ainsi d'identifier à la fois l'unité de prélèvement, l'outil de prélèvement, l'élément de prélèvement et l'élément de stockage. Ils peuvent également permettre de caractériser le prélèvement réalisé, en particulier l'horodate et le lieu du prélèvement, et le type de matériel animal prélevé.

Dans un mode particulier de réalisation, ces moyens d'identification peuvent être combinés avec des moyens d'identification d'une matière animale, par exemple pour permettre une traçabilité du prélèvement réalisé, ou d'au moins une partie de l'ensemble de prélèvement utilisé, avec la matière animale sur laquelle a été effectué le prélèvement.

L'ensemble de prélèvement 100 peut être conditionné dans un environnement stérile, par exemple un liquide ou un emballage stérile, pour former une unité de prélèvement, et être ainsi facilement transportable. Selon les modes de réalisation, l'emballage peut être hermétique, comme un sachet, ou se présenter sous forme de feuille, notamment d'un papier spécifique. En particulier, il peut s'agir d'un emballage perméable à certains gaz et rempli d'un gaz stérilisant après enveloppement de l'ensemble de prélèvement.

Dans un mode de réalisation particulier de l'unité de prélèvement, l'ensemble de prélèvement est ainsi enveloppé dans un tel emballage, puis placé dans une enceinte climatique, que l'on remplit ensuite d'oxyde d'éthylène afin d'obtenir un espace stérile autour de l'ensemble de prélèvement.

Dans d'autres modes de réalisation de l'unité de prélèvement, éventuellement complémentaires, l'ensemble de prélèvement peut être livré dans un emballage isotherme, hermétique ou destiné à le protéger des chocs.

Un tel emballage peut comprendre en outre d'autres moyens utiles au prélèvement, et notamment un ensemble de prélèvement supplémentaire et/ou au moins un élément de prélèvement ou de stockage supplémentaire, un réservoir de produit désinfectant, des moyens de traitement de l'échantillon permettant sa préparation, sa conservation, et/ou son traitement, ou encore un équipement visant une meilleure hygiène de l'utilisateur.

Ceci permet donc à l'utilisateur de disposer d'une unité de prélèvement clé en main, contenant à la fois le matériel nécessaire au prélèvement mais également d'autres moyens annexes comme un produit désinfectant ou des gants, ou des chapeaux jetables.

Dans une variante de réalisation, l'emballage peut également comprendre plusieurs ensembles de prélèvement, certains comportant par exemple des outils de prélèvement de formes différentes (par exemple de taille différente).

Ainsi, il est possible pour l'utilisateur de disposer de plusieurs outils à la fois et de choisir celui le mieux adapté en fonction par exemple du type d'animal à prélever, du prélèvement à effectuer, de la dangerosité de l'animal ....

### 5.2.5 Procédé de réalisation d'une unité de prélèvement

Différents modes de réalisation de l'unité de prélèvement peuvent être mis en oeuvre.

Ainsi, dans un mode de réalisation particulier, le procédé de réalisation de l'unité de prélèvement comprend :
- une étape de fabrication d'au moins un élément de prélèvement et/ou d'au moins un élément de stockage ;
- une étape de réalisation d'au moins un ensemble de prélèvement, au cours de laquelle au moins un desdits éléments de prélèvement et/ou desdits éléments de stockage est pré-monté sur un outil de prélèvement ;
- une étape de constitution de l'unité de prélèvement, au cours de laquelle l'ensemble pré-monté de prélèvement est conditionné dans un emballage.

Dans certains modes de réalisation, l'étape de réalisation de l'ensemble de prélèvement comprend, d'une part, une étape de fabrication de l'outil de prélèvement et, d'autre part, une étape de montage dudit élément de prélèvement et/ou dudit élément de stockage sur ledit outil de prélèvement.

Dans d'autres modes de fabrication, éventuellement complémentaires, l'étape de montage de l'élément de prélèvement et/ou de l'élément de stockage peut être effectuée au cours de l'étape de fabrication de l'outil. Ce dernier mode de réalisation est par exemple adapté à un mode de réalisation d'un outil de prélèvement à usage unique, où l'élément de prélèvement et/ou l'élément de stockage est enchâssé dans l'outil de prélèvement, de façon par exemple à ce qu'il soit impossible de retirer l'élément sans altérer l'outil.

L'élément de prélèvement et/ou l'élément de stockage peuvent être positionnés sur l'outil lors de l'étape de réalisation de l'ensemble de prélèvement indépendamment l'un de l'autre, ou associés.

Ainsi, dans le mode de réalisation particulier illustré en figure la, l'élément de prélèvement 120 est inséré lors de l'étape de réalisation de l'ensemble de prélèvement 100, dans l'élément de stockage 130. L'ensemble de prélèvement est ensuite emballé, pour former l'unité de prélèvement, lors de l'étape de constitution de l'unité de prélèvement.

Ce mode de réalisation permet d'éviter à un utilisateur de se blesser lors de la manipulation de l'élément de prélèvement et de protéger l'arête de l'élément de coupe de l'élément de prélèvement de l'environnement extérieur. Il permet également de protéger de l'air ambiant un agent spécifique éventuellement prévu dans la partie creuse de l'élément de prélèvement.

### 5.3 Présentation d'un second exemple

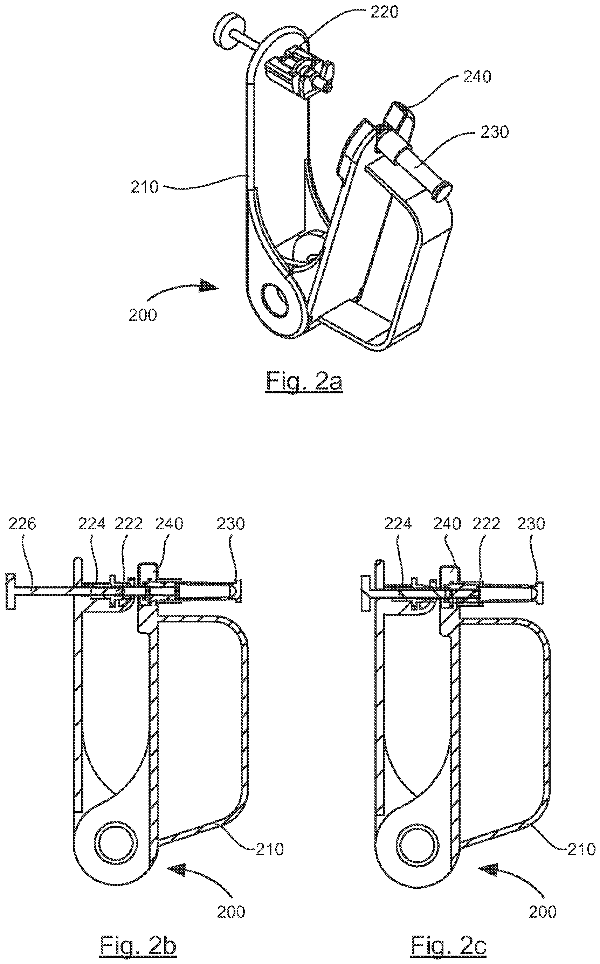

On présente en liaison avec les figures 2a à 2c, un second exemple d'ensemble de prélèvement 200 d'une unité de prélèvement, dans lequel on retrouve un élément de prélèvement 220, un élément de stockage 230, et un outil de prélèvement 210, comprenant au moins un support 240 de l'élément de stockage 230.

Selon ce mode de réalisation, l'élément de stockage 230 est pré-monté sur le support 240 avant que soit emballé l'ensemble de prélèvement pour former l'unité de prélèvement. L'élément de prélèvement 220 peut également être pré-monté sur un deuxième support, ou inséré dans l'élément de stockage 230 pour le transport jusqu'à l'utilisateur.

Dans ce mode de réalisation, comme illustré par les figures 2b et 2c, l'élément de prélèvement 220 peut comprendre, en sus de l'élément de coupe 222, qui jour un rôle similaire à l'élément de coupe 122 présenté en figure 2c, un élément poussoir 224, actionné par un pointeau 226.

Comme présenté sur les figures 2b et 2c, l'élément de coupe 222 est ouvert à ses deux extrémités, afin de laisser passer l'élément poussoir 224, de sorte que ce dernier puisse pousser l'échantillon de matière animale hors de l'élément de coupe 222 et l'accompagner dans l'élément de stockage 230, et venir obturer l'élément de stockage 230.

Une fois l'échantillon poussé dans l'élément de stockage, l'élément de prélèvement 220 utilisé peut, selon les modes de réalisation, être conservé avec l'élément de stockage ou jeté.

Dans une variante, le pointeau 226 peut de plus comprendre des moyens de retour, par exemple un ressort, afin de permettre un désengagement plus rapide et automatique de l'outil de la zone perforée et donc, dans le cas d'un prélèvement réalisé sur un animal vivant, une libération plus rapide de l'animal.

Comme le montre la figure 2a, la fermeture de l'outil est effectuée par rotation d'au moins une des deux branches de l'outil et confère donc un mouvement courbe à la trajectoire de l'élément de prélèvement. Ce premier mouvement de rotation permet à l'élément de coupe 222 de couper la matière animale. Dans un deuxième temps, le pointeau 226 est actionné selon un mouvement de translation, permettant de pousser l'élément poussoir 224 au travers de l'élément de coupe 226, comme décrit dans la demande de brevet FR-08 58453 précitée.

### 5.4 Présentation d'un troisième exemple

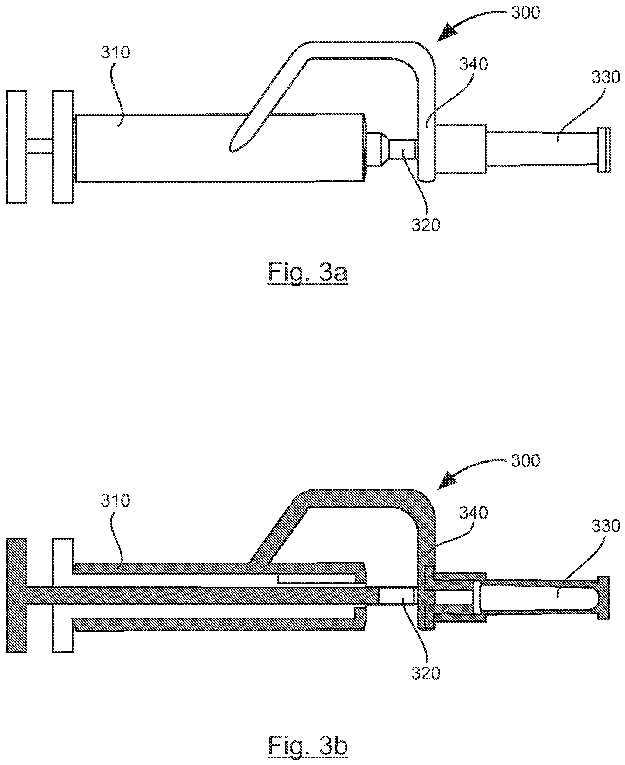

On présente en liaison avec les figures 3a et 3b, un troisième exemple d'un ensemble de prélèvement 300 d'une unité de prélèvement, dans lequel on retrouve un élément de prélèvement 320, par exemple une aiguille de biopsie, un élément de stockage 330, et un outil de prélèvement, par exemple une seringue 310, comprenant au moins un support 340.

La figure 3a présente une vue de face de l'ensemble de prélèvement 300 et la figure 3b une coupe transversale.

L'élément de stockage peut notamment être monté, de façon non amovible, dès la réalisation de l'ensemble de prélèvement 300 dans une position de prélèvement grâce au support 340.

Ce mode de réalisation permet d'assurer un usage unique de l'outil de prélèvement 310.

Dans ce mode de réalisation, l'outil 310 insuffle un mouvement de translation à l'élément de prélèvement 320, ce qui permet une meilleure précision du prélèvement. Une fois le prélèvement réalisé, l'élément de stockage peut être fermé par un bouchon ou par l'élément de prélèvement (après libération de l'oreille de l'animal par exemple), et la seringue transmise directement à un laboratoire pour analyse.

Dans une variante de ce mode de réalisation où l'élément de prélèvement peut être retiré du support 340, l'extrémité non destinée au prélèvement peut par exemple être terminée par un bouchon de façon à venir fermer l'élément de stockage.

### 5.5 Présentation d'un quatrième exemple

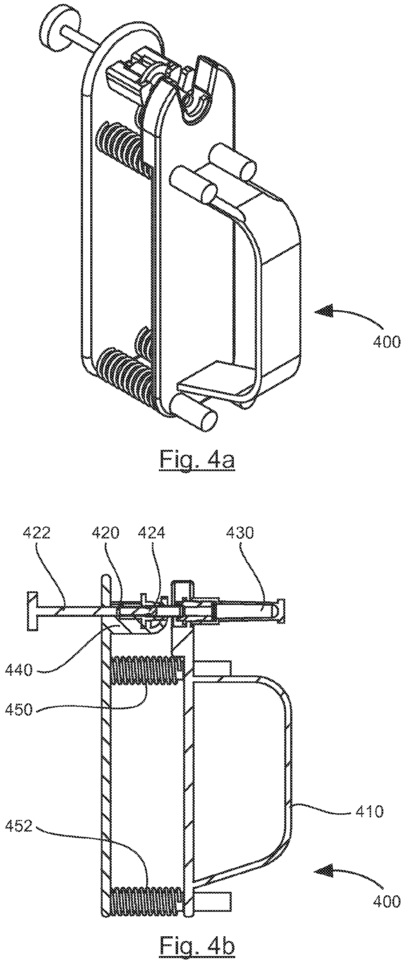

On présente en liaison avec les figures 4a et 4b, un quatrième exemple d'un ensemble de prélèvement 400 d'une unité de prélèvement, dans lequel on retrouve un élément de prélèvement 420, qui peut comprendre, en sus de l'élément de coupe 422, qui joue un rôle similaire à l'élément de coupe 122 présenté en figure 2c, un élément poussoir 424.

On retrouve également un élément de stockage 430, et un outil de prélèvement, par exemple une pince 410, comprenant au moins un support 440.

Selon ce mode de réalisation, l'élément de prélèvement 420 est pré-monté sur le support 440 avant que soit emballé l'ensemble de prélèvement pour former l'unité de prélèvement. L'élément de stockage 430 peut également être pré-monté sur un deuxième support, ou inséré sur l'élément de prélèvement jusqu'à l'utilisateur.

Par exemple, l'élément de stockage consiste en un tube de stockage tel que décrit par la demande de brevet FR-08 58453 précitée.

La figure 4a présente une vue en perspective de l'ensemble de prélèvement 400 et la figure 4b une vue en coupe.

Dans l'exemple illustré, le mécanisme de fermeture de la pince 410, et notamment les ressorts 450 et 452, insufflent un mouvement de translation à l'élément de prélèvement. De cette façon, la trajectoire de l'élément de coupe 422 et de l'élément poussoir 424 au travers du matériel animal à perforer est rectiligne, ce qui permet une meilleure précision du prélèvement.

Ce premier mouvement de translation permet à l'élément de coupe de couper la matière animale. Dans un deuxième temps, un pointeau 426 est actionné selon un deuxième mouvement de translation, permettant de pousser l'élément poussoir 424 au travers de l'élément de coupe 422.

Les figures 4a et 4b illustrent un exemple de réalisation mettant en oeuvre quatre ressorts. D'autres moyens de rappel, ou un nombre différent de ressorts (deux ressorts par exemple) peuvent également être envisagés.

### 5.6 Présentation d'un premier mode de réalisation de l'invention

On présente en liaison avec les figures 5a à 5d, un premier mode de réalisation d'un ensemble de prélèvement 500 d'une unité de prélèvement selon l'invention, dans lequel on retrouve un élément de prélèvement 520, un élément de stockage 530, composé de deux parties amovibles, par exemple un tube de stockage 532 et un bouchon 534, et un outil de prélèvement 510.

L'outil de prélèvement 510 comprend un premier support 540 destiné à recevoir l'élément de prélèvement associé à l'élément de stockage.

Selon l'invention, l'élément de prélèvement 520 est traversant, et est inséré au moins partiellement dans l'élément de stockage 530.

La figure 5a présente une vue de face de l'ensemble de prélèvement 500, la figure 5b une vue de dessus et les figures 5c et 5d des coupes transversales à des instants différents du prélèvement.

Comme illustré par ces figures, l'outil de prélèvement selon l'invention comprend une alvéole 550 sur laquelle vient s'appuyer l'élément de coupe de l'élément de prélèvement pour transpercer plus facilement la matière animale lors du prélèvement.

L'outil de prélèvement 510 porte également un bouchon 534, sur un support 542. Un tel bouchon comprend une tige permettant de repousser le matériel animal prélevé à travers l'élément de prélèvement, jusque dans l'élément de stockage 530.

L'outil de prélèvement est muni d'un axe de rotation à deux positions P1 et P2, qui permet de modifier la position de l'élément de prélèvement lors de la fermeture de la pince. Ainsi, une première position P1 de l'axe, met en face à face l'élément de prélèvement 520 et l'alvéole 550 pour la réalisation du prélèvement. Une seconde position P2 de l'axe met en face à face l'élément de prélèvement 520 et le bouchon 534 de façon à pousser l'échantillon de matière dans le tube de stockage 532 et à fermer l'extrémité de l'élément de prélèvement 520.

Une lame ressort, illustrée par la patte flexible 560, peut également être prévue.

Cette lame ressort permet notamment de repousser le matériel animal perforé, par exemple une oreille d'un animal, et de dégager l'élément de prélèvement du trou réalisé afin que l'élément de prélèvement ne reste pas « accroché » au matériel animal perforé, notamment à une oreille, et de faciliter le retour de l'outil de prélèvement dans sa position initiale une fois le prélèvement réalisé.

### 5.7 Présentation d'un deuxième mode de réalisation de l'invention

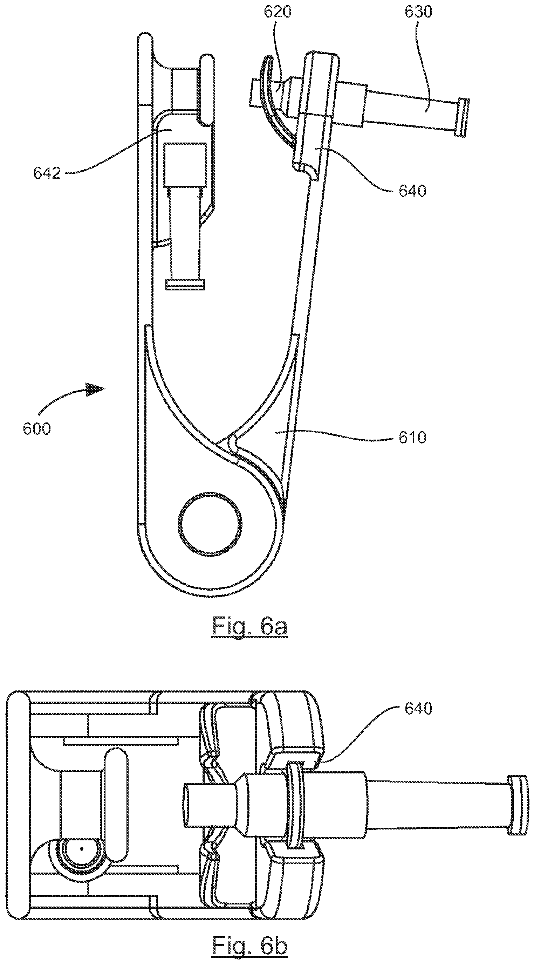

On présente un deuxième mode de réalisation d'un ensemble de prélèvement 600 d'une unité de prélèvement selon l'invention, en liaison avec les figures 6a et 6b qui en présentent respectivement une vue de profil et une vue de dessus.

Dans cet ensemble de prélèvement 600, on retrouve un élément de prélèvement 620, un élément de stockage 630 et un outil de prélèvement 610 comprenant un premier support 640 destiné à positionner l'élément de prélèvement, par exemple associé à l'élément de stockage, dans une position de prélèvement.

Selon l'invention, l'élément de prélèvement 620 est traversant, et est inséré au moins partiellement dans l'élément de stockage 630.

L'outil de prélèvement comprend deux branches, une première branche sur laquelle sont pré-montés l'élément de stockage et l'élément de prélèvement, et une deuxième branche comprenant une alvéole sur laquelle vient s'appuyer l'élément de coupe de l'élément de prélèvement pour transpercer plus facilement la matière animale lors du prélèvement.

L'outil de prélèvement 610 comprend également un second support 642. Ce second support peut définir une position initiale ou finale de transport pour l'élément de prélèvement et/ou l'élément de stockage

Selon une variante, ce second support permet le positionnement d'un second élément de stockage de secours.

Une lame ressort est également prévue. Elle permet notamment de repousser le matériel animal perforé, par exemple l'oreille d'un animal, et de dégager l'élément de prélèvement du trou réalisé, afin que l'élément de prélèvement ne reste pas « accroché » au matériel animal perforé, notamment à une oreille, et de faciliter le retour de l'outil de prélèvement dans sa position initiale une fois le prélèvement réalisé.

### 5. 8 Présentation d'un autre exemple

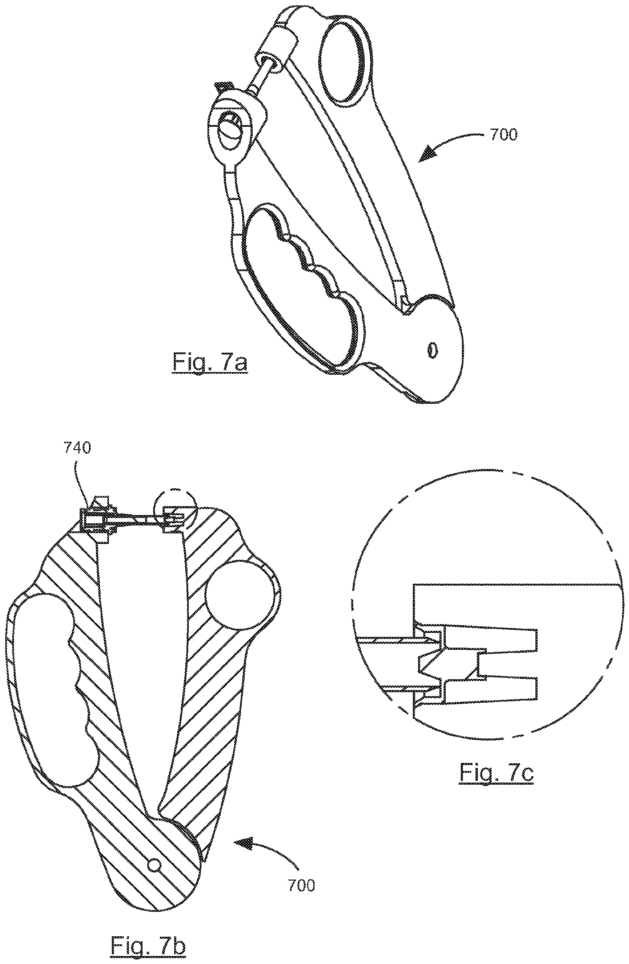

On présente un autre exemple d'un ensemble de prélèvement 700 d'une unité de prélèvement, en liaison avec les figures 7a à 7c qui en présentent une vue en perspective, une coupe transversale et l'extrémité ouverte de l'élément de prélèvement 720.

Dans cet ensemble de prélèvement 700, on retrouve un élément de prélèvement 720, et un outil de prélèvement 710 qui comprend un support 740 destiné à positionner l'élément de prélèvement 720 dans une position de prélèvement. Le support 740 est prévu sur une première mâchoire de l'outil. Une saillie prévue sur une deuxième mâchoire de l'outil permet de repousser la matière animale à l'intérieur de l'élément de prélèvement.

Ainsi, une fermeture partielle de l'outil de prélèvement permet la réalisation du prélèvement.

Une fermeture complète de l'outil (après retrait de l'oreille de l'animal par exemple) permet de pousser la matière animal à l'intérieur de l'élément de prélèvement.

L'élément de prélèvement peut ensuite être démonté de l'outil et inséré dans un élément de stockage non représenté.

Dans une variante, l'outil est transmis au laboratoire après fermeture complète. Dans ce cas, l'élément de prélèvement 720 peut également permettre le stockage du prélèvement réalisé. La fermeture complète de l'outil permet donc d'isoler l'élément de prélèvement une fois le prélèvement réalisé.

Dans une variante de ce mode de réalisation, l'élément de prélèvement 720, qui joue également le rôle d'élément de stockage du prélèvement réalisé, est solidarisé de façon irréversible à l'outil de prélèvement 710 par l'intermédiaire du support 740.

### 5.9 Autres modes de réalisation

Les modes de réalisation de l'invention ne se limitent pas aux modes de réalisation présentés ici. D'autres modes de réalisation, intuitifs à un homme de métier à la lecture de la description de l'invention, sont possibles. Par exemple, des modes de réalisation présentés, ou des options relatives à ces modes de réalisation, comme la présence de plusieurs supports ou de moyens de retour, peuvent en particulier être combinés entre eux.

Dans une variante de l'invention, l'outil de prélèvement comprend des moyens de fermeture de l'élément de stockage. Il peut s'agir par exemple d'un simple bouchon comme illustré en figures 5a à 5d, ou de moyens empêchant l'accès à l'élément de stockage, comme par exemple des moyens permettant de le sceller ou de sceller l'outil lui même si l'élément est porté sur un support de l'outil dans une position finale de transport.

De cette façon, une fois le prélèvement réalisé, il est possible d'envoyer directement l'outil de prélèvement à un laboratoire, en vu du retrait et de l'analyse du matériel animal. Cette opération est possible du fait de l'usage unique auquel est destiné l'ensemble de prélèvement d'une unité de prélèvement selon l'invention.

Selon un autre mode de réalisation, l'outil de l'ensemble de prélèvement présenté pourrait être couplé avec un autre dispositif, ou posséder des moyens complémentaires, permettant notamment la mise en place consécutive ou simultanée d'une marque d'identification animale. En particulier, cette marque d'identification peut porter un identifiant lié à un moyen d'identification de l'ensemble de prélèvement et/ou de l'unité de prélèvement.

De plus, une variante de l'invention prévoit que l'outil de prélèvement puisse être installé sur un autre dispositif, par exemple un dispositif de marquage, par le biais duquel il est actionné.

Dans tous les modes de réalisation présentés, l'ensemble de prélèvement est destiné à un usage unique, et pourrait donc être jeté, et en particulier au moins partiellement recyclé, après une seule utilisation.

En effet, l'outil n'est pas nécessairement réalisé en aluminium ou en acier, mais peut être réalisé en plastique, ou un autre matériau léger, ne présentant pas la même longévité que les outils de prélèvement de l'art antérieur.

Dans certains modes de réalisation, l'outil de prélèvement est conçu de façon être quasiment plat, ou de faible épaisseur afin de faciliter son transport et notamment son envoi par voie postale, avant ou après le prélèvement.

Le faible coût de réalisation de l'outil de prélèvement permet en effet de proposer un outil jetable.

Dans tous les modes de réalisation présentés, l'emballage de l'unité de prélèvement contenant l'ensemble de prélèvement est susceptible d'être stérile, et notamment hermétique ou permettant une protection contre les chocs, par exemple un sachet matelassé adapté pour un envoi par voie postale. Il peut aussi s'agir d'un emballage stérile sous forme d'au moins une feuille enveloppant l'ensemble de prélèvement et remplie d'un gaz stérilisant. L'emballage lui-même peut être recyclable ou prévu pour être réutilisé, notamment pour le transport de l'élément de stockage une fois le prélèvement réalisé.

## Revendications

1. Unité de prélèvement d'au moins un échantillon de matière animale, formée d'un emballage comprenant au moins un ensemble de prélèvement (600) comprenant :
- une pince de prélèvement (610) comprenant une première branche et une deuxième branche ;
- un élément de prélèvement (620) dudit échantillon, ledit élément de prélèvement comprenant un élément de coupe ;
- un élément de stockage (630) dudit échantillon ; **caractérisée en ce que** :
- la première branche comprend un premier support (640) sur lequel est pré-monté l'élément de stockage (630) et l'élément de prélèvement (620), dans l'emballage, ledit premier support (640) étant configuré pour positionner ledit élément de prélèvement (620) dans une position de prélèvement,
- la deuxième branche comprend une alvéole configurée pour servir d'appui à l'élément de coupe dudit élément de prélèvement (630) de sorte à perforer ladite matière animale lors du prélèvement,
**en ce que** ladite pince de prélèvement (610) comprend également
- un moyen de retour configuré pour repousser ladite matière animale perforée et pour ramener ladite pince de prélèvement (610) dans une position initiale une fois le prélèvement réalisé,
et **en ce que** ledit élément de prélèvement (620) est traversant et inséré au moins partiellement dans l'élément de stockage (630).

2. Unité de prélèvement selon la revendication selon la revendication 1, **caractérisée en ce que** ledit élément de prélèvement (620) est pré-monté sur ledit premier support (640) dans ladite position de prélèvement, de façon irréversible.

3. Unité de prélèvement selon l'une quelconque des revendications 1 et 2, **caractérisée en ce que** ledit élément de prélèvement (620) est logé de façon amovible dans ledit élément de stockage (630).

4. Unité de prélèvement selon l'une quelconque des revendications 1 à 3, **caractérisée en ce qu'**au moins un desdits éléments de prélèvement (620) et/ou de stockage (630) contient au moins un agent spécifique appartenant au groupe comprenant :
- un agent désinfectant ;
- un agent cicatrisant ;
- un agent conservateur ;
- un agent dessiccant ;
- un médicament ;
- un vaccin ;
- une combinaison d'au moins deux des agents précédents.

5. Unité de prélèvement selon l'une quelconque des revendications 1 à 4, **caractérisée en ce que** ladite pince de prélèvement (610) comprend des moyens de fermeture dudit élément de stockage (630).

6. Unité de prélèvement selon l'une quelconque des revendications 1 à 5, **caractérisée en ce que** ladite pince de prélèvement (610) comprend au moins un second support (642), et **en ce que** ledit au moins un second support (640, 642) définit une position finale de transport pour l'élément de prélèvement (620) et/ou l'élément de stockage (630) ou est destiné à porter au moins un élément de prélèvement et/ou de stockage supplémentaire.

7. Unité de prélèvement selon l'une quelconque des revendications 1 à 6, **caractérisée en ce que** ladite pince de prélèvement (610) est, au moins partiellement, recyclable.

8. Unité de prélèvement selon l'une quelconque des revendications 1 à 7, **caractérisée en ce que** ladite pince de prélèvement (610) est, au moins partiellement, conçu dans un matériau permettant un usage unique de ladite pince.

9. Unité de prélèvement selon l'une quelconque des revendications 1 à 8, **caractérisée en ce que** ladite pince de prélèvement (610), ledit élément de stockage (630), ledit élément de prélèvement (620) et/ou ledit emballage comprennent des moyens d'identification permettant d'identifier ledit ensemble de prélèvement et/ou ladite unité de prélèvement.

10. Unité de prélèvement selon l'une quelconque des revendications 1 à 9, **caractérisée en ce que** ledit emballage est stérile.

11. Unité de prélèvement selon l'une quelconque des revendications 1 à 10, **caractérisée en ce que** ledit emballage comprend en outre au moins un élément de prélèvement (620) ou de stockage (630) supplémentaire.

12. Unité de prélèvement selon la revendication 1, **caractérisé en ce que** ledit moyen de retour est une lame ressort.

## Patentansprüche

1. Einheit zur Entnahme von zumindest einer Probe von tierischem Material, die durch eine Packung ausgebildet ist, die zumindest eine Anordnung zur Entnahme (600) aufweist, die aufweist:
- eine Entnahmeklemme (610), die einen ersten Schenkel und einen zweiten Schenkel aufweist;
- ein Element zur Entnahme (620) der Probe, wobei das Element zur Entnahme ein Schneidelement aufweist;
- ein Element zur Lagerung (630) der Probe;
**dadurch gekennzeichnet, dass**:
- der erste Schenkel einen ersten Träger (640) aufweist, an welchem das Element zur Lagerung (630) und das Element zur Entnahme (620) vormontiert ist, wobei der erste Träger (640) in der Packung dazu eingerichtet ist, das Element zur Entnahme (620) in einer Entnahmeposition zu positionieren,
- der zweite Schenkel eine Buchse aufweist, die dazu eingerichtet ist, als Auflager für das Schneidelement des Elements zur Entnahme (630) zu dienen, wodurch das tierische Material während der Entnahme perforiert wird,
dadurch, dass die Entnahmeklemme (610) ebenfalls ein Rückholmittel aufweist, das dazu eingerichtet ist, das perforierte, tierische Material auszustoßen und die Entnahmeklemme (610) nach erfolgter Entnahme in eine Anfangsposition zurückzubringen,
und dadurch, dass das Element zur Entnahme (620) durchgehend und zumindest teilweise in dem Element zur Lagerung (630) eingeschoben ist.

2. Einheit zur Entnahme nach Anspruch 1, **dadurch gekennzeichnet, dass** das Element zur Entnahme (620) an dem ersten Träger (640) in der Entnahmeposition vormontiert ist, auf eine irreversible Art und Weise.

3. Einheit zur Entnahme nach einem der Ansprüche 1 und 2, **dadurch gekennzeichnet, dass** das Element zur Entnahme (620) herausnehmbar in dem Element zur Lagerung (630) aufgenommen ist.

4. Einheit zur Entnahme nach einem der Ansprüche 1 bis 3, **dadurch gekennzeichnet, dass** zumindest eines der Elemente zur Entnahme (620) und/oder zur Lagerung (630) zumindest ein spezifisches Mittel aufweist, das zu der Gruppe gehört, die aufweist:
- ein Desinfektionsmittel;
- ein Wund- und Heilmittel;
- ein Konservierungsmittel;
- ein Trockenmittel;
- ein Medikament;
- ein Impfstoff;
- eine Kombination von zumindest zwei der vorangegangenen Mittel.

5. Einheit zur Entnahme nach einem der Ansprüche 1 bis 4, **dadurch gekennzeichnet, dass** die Entnahmeklemme (610) Verschlussmittel für das Element zur Lagerung (630) aufweist.

6. Einheit zur Entnahme nach einem der Ansprüche 1 bis 5, **dadurch gekennzeichnet, dass** die Entnahmeklemme (610) zumindest einen zweiten Träger (642) aufweist, und dass der zumindest zweite Träger (640, 642) eine Transportendposition für das Element zur Entnahme (620) und/oder das Element zur Lagerung (630) definiert oder dazu bestimmt ist, zumindest ein Element zur Entnahme und/oder zur zusätzlichen Lagerung zu tragen.

7. Einheit zur Entnahme nach einem der Ansprüche 1 bis 6, **dadurch gekennzeichnet, dass** die Entnahmeklemme (610) zumindest teilweise recycelbar ist.

8. Einheit zur Entnahme nach einem der Ansprüche 1 bis 7, **dadurch gekennzeichnet, dass** die Entnahmeklemme (610) zumindest teilweise aus einem Material ausgebildet ist, das eine einmalige Benutzung der Klemme ermöglicht.

9. Einheit zur Entnahme nach einem der Ansprüche 1 bis 8, **dadurch gekennzeichnet, dass** die Entnahmeklemme (610), das Element zur Lagerung (630), das Element zur Entnahme (620) und/oder die Packung Identifikationsmittel aufweisen, die es ermöglichen, die Anordnung zur Entnahme und/oder die Einheit zur Entnahme zu identifizieren.

10. Einheit zur Entnahme nach einem der Ansprüche 1 bis 9, **dadurch gekennzeichnet, dass** die Packung steril ist.

11. Einheit zur Entnahme nach einem der Ansprüche 1 bis 10, **dadurch gekennzeichnet, dass** die Packung außerdem zumindest ein Element zur Entnahme (620) oder zur zusätzlichen Lagerung (630) aufweist.

12. Einheit zur Entnahme nach Anspruch 1, **dadurch gekennzeichnet, dass** das Rückholmittel eine Blattfeder ist.

## Claims

1. Unit for collecting at least one sample of animal material, formed by a package comprising at least one collecting set (600) comprising:
- a collecting set of pliers (610) comprising a first arm and a second arm;
- a collecting component (620) for collecting said sample, said collecting component comprising a cutting component;
- a storage component (630) for storing said sample; **characterized in that**:
- the first arm comprises a first support (640) on which the storage component (630) and the collecting component (620) is pre-mounted, said first support (640) being configured to position said collecting component (620) in a collecting position,
said second arm comprises a recess configured to act as a support for the cutting component of the collecting component (630), in order to perforate said animal material during the collection,
**in that** the collecting set of pliers (610) also comprises
- a return means configured to push back the perforated animal material and to bring back the collecting set of pliers (610) into an initial position once the collection is done,
and **in that** said collecting component (620) is through and inserted at least partially in said storage component (630).

2. Collecting unit according to claim 1, **characterized in that** said collecting component (620) is pre-mounted on said first support (640) in said collecting position, irreversibly.

3. Collecting unit according to any one of the claims 1 and 2, **characterized in that** said collecting component (620) is housed detachably in said storage component (630).

4. Collecting unit according to any one of the claims 1 to 3, **characterized in that** at least one of said collecting (620) and/or storage (630) components contains at least one specific agent belonging to the group comprising:
- a disinfectant;
- a cicatrizing agent;
- a preserving agent;
- a desiccant;
- a medicine;
- a vaccine;
- a combination of at least two of the above agents.

5. Collecting unit according to any one of the claims 1 to 4, **characterized in that** said collecting set of pliers (610) comprises means for closing said storage component (630).

6. Collecting unit according to any one of the claims 1 to 5, **characterized in that** said collecting set of pliers (610) comprises at least one second support (642) and **in that** said at least one second (640, 642) defines a final position of transport for the collecting component (620) and/or the storage component (630), or is intended for carrying at least one additional collecting and/or storage component.

7. Collecting unit according to any one of the claims 1 to 6, **characterized in that** said collecting set of pliers (610) is at least partially recyclable.

8. Collecting unit according to any one of the claims 1 to 7, **characterized in that** said collecting set of pliers (610) is at least partially designed in a material enabling a one-time use of said set of pliers.

9. Collecting unit according to any one of the claims 1 to 8, **characterized in that** said collecting set of pliers (610), said storage component (630), said collecting component (620) and/or said package comprise means of identification enabling said collecting set and/or said collecting unit to be identified.

10. Collecting unit according to any one of the claims 1 to 9, **characterized in that** said package is sterile.

11. Collecting unit according to any one of the claims 1 to 10, **characterized in that** said package furthermore comprises an additional collecting (620) or storage (630) component.

12. Collecting unit according to claim 1, **characterized in that** said return means is a leaf spring.
